# EUROPEAN PATENT SPECIFICATION

(11) **EP 2 208 498 B1**
(45) Date of publication and mention of the grant of the patent: **26.02.2014**
(21) Application number: 08829371.7
(22) Date of filing: 05.09.2008
(51) Int. Cl.: A61K 45/06, A61K 31/185, A61K 31/7048, A61P 7/02

(54) **PHARMACEUTICAL COMPOSITION COMBINING VARIOUS VENOTONIC AND VASOPROTECTIVE AGENTS FOR THE TREATMENT OF CHRONIC VENOUS INSUFFICIENCY**
PHARMAZEUTISCHE ZUSAMMENSETZUNG AUS EINER KOMBINATION VON VERSCHIEDENEN VENOTONISCHEN UND VASOPROTEKTIVEN MITTELN ZUR BEHANDLUNG VON CHRONISCHER VENENINSUFFIZIENZ
COMPOSITION PHARMACEUTIQUE COMPRENANT UNE COMBINAISON DE DIVERS AGENTS VEINOTONIQUES ET VASOPROTECTEURS POUR LE TRAITEMENT DE L'INSUFFISANCE VEINEUSE CHRONIQUE

(30) Priority: 07.09.2007 MX 2007011002
(43) Date of publication of application: 21.07.2010
(73) Proprietor: World Trade Import-Export, WTIE A.G., 6302 Zug (CH)
(72) Inventor: GARCÍA ARMENTA, María Elena, C.P. 45020 Guadalajara Jalisco (MX); SANTOS MURILLO, Josefina, C.P. 44600 Zapopan Jalisco (MX); ÁLVAREZ OCHOA, Victor Guillermo, C.P. 45222 Zapopan Jalisco (MX)
(74) Representative: Carvajal y Urquijo, Isabel
(86) International application number: PCT/MX2008/000119
(87) International publication number: WO 2009/031878

(56) References cited:
- EP-A1- 0 711 560
- DATABASE BIOSIS [Online] BIOSCIENCES INFORMATION SERVICE, PHILADELPHIA, PA, US; March 2004 (2004-03), CIAPPONI AGUSTIN ET AL: "Calcium dobesilate for chronic venous insufficiency: A systematic review." XP002603117 Database accession no. PREV200400215889 & ANGIOLOGY, vol. 55, no. 2, March 2004 (2004-03), pages 147-154, ISSN: 0003-3197
- MISRA M.C. ET AL.: 'Drug treatment of haemorrhoids' DRUGS vol. 65, no. 11, 2005, pages 1481 - 1491, XP008124604
- LYSENG-WILLIAMSON ET AL.: 'Micronised purified flavonoid fraction.A review of its use in chronic venous insufficiency,venous ulcers and haemorrhoids' DRUGS vol. 63, no. 1, 2003, pages 71 - 100, XP008132623
- SARABIA M. ET AL.: 'Calcium dobesilate versus purified flavonoid fraction of diosmin in the treatment of haemorrhoidal crises: a randomized,controlled study with an initial double-blind,double-dummy period' CURRENT THERAPEUTIC RESEARCH vol. 62, no. 7, 2001, pages 424 - 529, XP003026587
- STRUCKMANN J.R.: 'Clinical efficiacy of micronized purified flavonoid fraction:an overview' JOURNAL OF VASCULAR RESEARCH vol. 36, no. 1, 1999, pages 37 - 41, XP008127886
- MARTINEZ-ZAPATA M.J.: 'A randomized,double-blind multicentre clinical trial comparing the efficiacy of calcium dobesilate with placebo in the treatment of chronic venous disease' EUROPEAN JOURNAL VASCULAR ENDOVASCULAR SURGERY vol. 35, 2008, pages 358 - 365, XP022523507

## Description

### FIELD OF INVENTION

The present invention has application in pharmaceutical industry and describes a pharmaceutical composition comprising the synergistic combination of three agents which manifest pharmacological activity as venotonic and vasoprotective, such as active principles: Diosmine, Hesperidin and Calcium dobesilate, in addition to pharmaceutically acceptable excipients; which are formulated in a single dosage unit to be orally administered, indicated for control and treatment of chronic venous insufficiency and related diseases.

### BACKGROUND OF INVENTION

The combination of above mentioned active principles produces a larger synergistic effect when administered in combination in a single dosage unit, unlike when they are independently administered, thus causing benefits such as: lower concentration delivery of active principles comprised in the formula, lower administered doses, faster pharmacological action, enhancement of therapeutic effect and lower risks of showing side effects.

There are two differentiated venous systems in lower limbs: surface (SVS) and deep (SVP), joined by perforating or communicating veins. Surface system veins have some thinner walls which are surrounded by easily distended tissues, they are distributed in a network shape and present a great individual variability in location. The deep system harbors 90% of venous blood in lower limbs and shows thicker walls and a less distension capability.

Veins from lower limbs are responsible for the blood to go into heart, whereby they have a faced semilunar valve system which makes that blood flow moves upwardly and in a centripetal way (from SVS to SVP). Moreover, in order to move blood against gravity, leg muscle contraction acts as a pump squeezing the surrounding veins.

Chronic venous insufficiency (CVI) is a state wherein venous return is difficult, particularly in bipedalism when venous blood flows in an opposite direction to normal one (from SVP to SVS).

Varices are veins which show permanent and pathologic dilatations, with extension and flexuosity. They mostly appear in lower limbs. They are determined as the visible face from CVI.

Chronic venous insufficiency is the most frequent vascular disease, affecting between 20 - 30% from adult population and 50% of those older than 50 years. It is 5-fold more frequent in women.

Determining factor of CVI appearances is venous valve incompetency, a situation reached whether by a destruction thereof (traumatism, thrombus rechanneling) or by an idiopathic defect in venous wall caused by a vein excessive dilatation and valve separation.

Vascular insufficiency favors blood circulation from deep to surface system, causing an increase in hydrodynamic pressure therein, with a latter appearance of varices. This venous' hypertension damages microcirculation and increases post-capillary vessel transmural pressure, with liquid exudation, edema and bad surrounding tissue oxygenation accordingly.

All these changes favor inflammation, infection, thrombosis and tissular necrosis, thus leading to disease complications.

Main risk factors associated with varices presence are: a) Age, the older the higher risk; b) Parity; c) Prolonged bipedalism; d) Obesity, only in women, not being a risk factor in men.

Chronic venous insufficiency is manifested with one or two of the following symptoms: heaviness, pain, itch, tiredness, muscular cramps and swelling in lower limbs which worsens with orthostatism or heat and improves with decubitus or cold.

Symptom severity does not correspond with varices extension or size, neither with the reflux volume, and many of these symptoms are present in persons without venous pathology.

Moreover, there are differences between sexes as to symptomatology being related the presence of trunk varices in women with itch, heaviness and pain, while in men is only related with itch.

In women, symptoms may worsen with menstruation, pregnancy and with oral contraceptives or sustitute hormonal treatments. Complications which are more frequently observed are: a) Cutaneous: pigmentation (dermatitis ocre), hemorrhage; varicose eccema, surface thrombophlebitis, hypodermitis, deep venous thrombosis; cellulitis, lymphangitis; phlebostatic ulcer. b) Vascular: hemorrhage; varicose eccema, surface thrombophlebitis, hypodermitis, deep venous thrombosis; cellulitis, lymphangitis.

From the morphological point, varices are classified as:
a) Telangiectasies or vascular spiders: they are dilatations of small veins or intradermal capillaries.
b) Reticular varices: they are dilatations of small gauge veins, generally in thigh outer face, leg, knee and in popliteus void.
c) Trunk varices: they affect saphenous veins or affluent branches.

As to clinic, there is a number of classifications (Widmer, Porter) but CEAP classification is discussed, which is recommended by the Spanish Society of Angiology and Vascular Surgery.

C letter assesses clinical findings: C0= No venous injury visible or palpable signs, C1= presence of telangiectasy or reticular veins, C2= varices, C3= edema, C4= cutaneous changes related with a venous pathology without ulcers, C5= cutaneous changes with healed ulcer, C6= cutaneous changes with active ulcer. "A" letter is written after the number when patient is asymptomatic and "S" when symptoms are present. "E" letter refers to etiology: Ec= congenital disease, Ep=primary disease or without known cause, Es= secondary disease or with known cause. "A" letter describes anatomical findings found with Echo-Doppler: Surface veins (As)*, deep veins (Ap)* and perforating veins * (* A number is added as a function of affected vein). "P" letter makes reference to physiopathology: PR= reflux, PO= obstruction, PR, O= both. Moreover, a scale shall be used which measures impairment caused by CVI: 0=asymptomatic patient, 1= patient with symptoms not requiring compression measures, 2= patient who may work 8 hours only with compression measures, 3= patient unable to work even with compression measures.

Certain measures shall be taken for treatment of CVI such as: a) Weight reduction in case of obesity; b) Preventing prolonged bipedalism or sitting position periods; c) Use of wide and comfortable footwear, preventing the use of heels; d) Performing exercises which involve axle flexion, for activating muscle pumping; e) Foot rise over heart level during 15-30 minutes several times a day, for symptomatology reduction and edema. Sleeping with feet in bed raised about 15 cm. , f) Preventing heat.

Compression improves venous return and reduces reflux, decreasing venous pressure. It improves symptomatology and edema, retarding disease evolution. It is prescribed in all patients showing CVI or varices symptomatology and having an ankle/arm index > 0.9.

Elastic compression stockings apply a decreasing pressure from ankle to waist or knee. They are classified according to the pressure applied over malleollus, in light-mild compression stockings, normal compression and strong compression. Prescription for each class keeps a direct relationship with disease severity, the larger complication severity or symptomatology the higher pressure that shall be applied.

It is important to adapt the stocking size for every patient by following the indications from every manufacturer, in order to prevent that the stocking acts as a tourniquet by being too tightened or being inefficient for not applying sufficient pressure. Moreover, patient shall be warned that stockings shall be used before waking up bed, the time when veins are less filled.

Most patients will have a good response to the use of short stockings (up to knee) but for those having thigh symptomatic varices, the use of high stockings is prescribed.

Inelastic compression is based in the creation of a rigid wrapping around the leg in such a way that provides pressure during muscle contracture phase, but not in rest, which allows a better venous emptying. It is more efficient than elastic compression in cases of more severity (venous ulcer, adverse symptomatology to another treatment). This compression type may be applicable through: a) Dressings: their efficacy depend on skill upon arrangement; b) "Unna sleeve": it is a cotton garment which is stretched from ankle to knee, attached with laces in front side which allows pressure adjustment; c) "Circ-Aid": a series of nylon strips surrounding the leg and adjusted around; d) Pneumatic compression pumps, which are prescribed for lymphedema, venous ulcers, or the presence of severe edema. They shall be used associated with another type of compression measures.

Pharmacologic therapy includes venotonic and vasoprotective drugs which improve CVI symptomatology.

Currently, most of drugs which are found in marketplace for treatment of chronic venous insufficiency comprise active principles which are independently formulated, which fulfill with a so specific therapeutic activity, which in many times is limited as to symptom diversity showed by patients who suffer said disease.

SARABIA M. ET AL.: "Calcium dobesilate versus purified flavonoid fraction of diosmin in the treatment of haemorrhoidal crises: a randomized,controlled study with an initial double-blind,double-dummy period",CURRENT THERAPEUTIC RESEARCH, vol. 62, no. 7, 2001, pages 424-529, describes a study that compares the efficacy and tolerance of calcium dobesilate and micronized flavonoid fraction of diosmine (90% diosmin and 10% flavonoids expressed as hesperidin) in the treatment of haemorrhoidal crises. 51 patients were treated, 25 received 1 500 mg per day of calcium dobesilate per day, and 26 received 3000 mg of flavonoid fraction of diosmine per day; both groups experienced a significant improvement.

### SUMMARY OF INVENTION

There are several drug groups used in this pathology which constitute a drug bank which when duly prescribed, they provide excellent results in venolymphatic insufficiency conditions and their complications.

Development of present invention was carried out in order to provide a useful pharmaceutical alternative for control and treatment of chronic venous insufficiency by reducing symptomatology and improving venous return, in addition to decrease the risk of showing complications, whereby a pharmaceutical composition comprising a combination of several.venotonic and vasoprotective agents is described, those which act synergically and formulated in a single dosage unit to be orally administered, providing significant benefits such as: lower concentrations of active principles comprised in the formula, lower administered doses, enhancement of therapeutic effect, faster pharmacological action, risk reduction in presenting complications and a decrease in risk of manifesting secondary effects.

### DETAILED DESCRIPTION OF INVENTION

Venotonic or vasoprotective agents are a broad group of drugs acting over smooth muscle fibers from venous wall, through calcium-dependant mechanisms modulated by an alpha-adrenergic neurohormonal regulation. Venous tone increase is by alpha-adrenergic receptor stimulation or by noradrenaline degradation inhibition. Its use does not replace but supplements instead the compression measures and posture care.

Venotonic agents reduce fragility and vascular permeability, in addition to partially improving symptomatology, especially tiredness sensation and edema. Many of them have their origin in rutoside, a glucoside attributed with vitaminic properties being exemplary: chromocarb, Diosmine, flavodic acid, hidrosmine and troxerutine. Other drugs develop additional antihemorrhagic effects such as: aminaftone and naftazone. Calcium dobesilate, horse-chestnut extract and its main active ingredient, escine are also classified as venotonics.

The use of venotonic agents in patients who suffer chronic venous insufficiency has the purpose of temporarily reducing the intensity of symptoms manifested by said pathology.

It is important to remind the existence of a series of useful measures for decreasing venous stasis such as: moderate physical exercise practice (walking, pedaling, swimming), and no high-heeled footwear, in addition to elastic stockings above mentioned.

Anticoagulant efficacy in a number of indications is accepted by everybody and is no longer discussed. That also, occurs with fibrinolytics and antiinflammatories. However, indications for antiaggregation agents, diuretics and several topical preparations may be a matter of discussion. Sclerotizing technique requires the use of a series of substances which may be considered as drugs. There are discussions from time to time about the convenience of using or not venotonics and lymphotropic drugs. Physiopathologic objectives of treatment with venolymphotropic agents are mainly: increase in venous tone, capillary permeability reduction and capillary fragility decrease.

Particularly the effects over venous tone and capillary permeability have been those with more study, constituting two of the axis of therapeutic research over efficacy of several drugs. More recently and according to advancements in exploration methods and physiopathologic knowledge, three objectives have been established: correction of microcirculatory disturbances; correction of hemorrheological deviations; improvement of parietal fibrinolytic activity.

Venotonic and lymphotropic agent efficacy and safety has been manifested in a number of double-blind works in front of placebo and three years of clinical experience in their use. Thus, we may consider them as useful for treatment of venolymphatic insufficiency. An advise against its use is a severe mistake and assumes a derivation towards other less suitable drugs in venolymphatic pathology, with the consequent negative effect in evolution of these processes, which is evident in an increase of ambulatory visits, an increase in hospital income and higher labor absences.

Vascular pathology has been benefited a lot in the last decades from a higher development in pharmacological research. Thus, active principles have appeared which directly affect vessel wall or dynamics, drugs which are interrupted by coagulation cascade or lymphovenous syndromic feature modificators, such as edema or inflammation, have improved the safety and prognosis in managing this pathology in their more recent versions.

Oral medication which is very similar to that currently used was already described in Ebers's papyrus (1500 B.C). In spite of elapsed time, there have been limited advancements in this field. More than 60% of drugs which are used daily proceed or have their origin in vascular plants, algae and fungi. Many phlebology professionals even ignore the natural origin of many of the drugs in use.

Pharmacokinetic studies have demonstrated that drug excretion and absorption velocity and magnitude are more important than dose for determining their action over body. There has been a remarkable progress in threapeutic system development which allows targeting drugs towards predetermined cells or organs. Final objective of any active principle development process consists of collecting sufficient information to prepare a risk/profit balance about its use in a specific clinical condition in terms of comparing several alternatives, in addition to prevent adverse reactions which cause unfavorable and unintentional changes in structure (signs), function (symptoms) or chemistry (laboratory data) in the body, temporarily associated with the use of a drug in human beings.

Venoactive drugs include the following: a) venous tonic such as *Ruscus aculeatus, Aesculus hippocastanum, Brusco, Esculina*; b) drugs which increase microvascular resistance such as anthocyanosides, Calcium dobesilate, Diosmine, Hesperidin, chromo-carbo-diethylamine, rutine, troxerrutine and hydroxy-ethyl-rutoside; c) lysosome enxyme blockers such as naftasone; d) venolymphatic action drugs such as hidrosmine, micronized diosmine and e) lymphothrope drugs such as lymph flow activators such as *Melilotus officinalis, Ruscus aculeatus,* Troxerrutine, Hydroxy-ethyl-rutosides.

Micronized Purified Flavonoid Fraction comprises 90% Diosmine and 10% flavonoids expressed as Hesperidin; those which belong to the venolymphatic action drug group, which protects microcirculation from secondary damage and venous pressure increased by walking.

Micronized Purified Flavonoid Fraction reduces the leukocyte and endothelial cell interaction, inhibiting the intercelular endothelial adhesion I molecule (ICAM-I) and vascular cell adhesion molecule (VCAM), as well as the expresson on the surface of some leukocyte adhesion molecules (monocytes or neutrophils CD62 L, CD11B).

Diosmine and Hesperidin exert their action over return vascular system. At vein level they decrease venous distension and reduce venous stasis. At microcirculation level, they normalize capillary permeability and reinforce capillary resistance.

They produce an increase in venous tone since they extend noradrenaline activity over venous wall, they not only activate venous return but they also stimulate and improve lymphatic drainage, improving microcirculation, protecting the venous wall against the biochemical mediators involved in inflammatory reaction and exerting an inhibiting effect over PGE₂, PGF₂ α and Thromboxane B₂ prostaglandin synthesis at the same time acting over oxygen reactive forms produced by polymorphonuclear and inhibiting cyclic AMP degradation reaction by phosphodiesterase action. They increase venous tone since they act directly and powerfully over the tunica muscularis on venous wall, thus improving venous debit, decreasing venous distress and stasis.

Calcium dobesilate (2,5 dihydroxy-benzene-sulfonate), different from plant extracts, has a precise chemical structure and well defined pharmacological properties. It acts over endothelium and capillary basement membrane, blocking hyperpermeability, inhibiting platelet aggregation and inhibiting blood hyperviscosity and improving lymphatic drainage.

Calcium dobesilate inhibits vasoactive substances responsible of endothelial cell contracture or shortage; bradykinin, histamine and serotonin, normalizing capillary permeability.

It inhibits enzymes which degrade mucopolysaccharides, components of basement membrane, and eases basement membrane collagenoid substance cross-linkage by calcium dobesilate - collagen covalent binds, thus normalizing capillary resistance.

Calcium dobesilate prevents platelet membrane destruction and thrombogeneic activity,- further manifesting an erythrocitary antiagglutinant effect which produces an improvement in blood viscosity; increasing ellasticity and erythrocitary deformation, allowing a better tissue irrigation; reducing high density plasma protein level, decreasing plasma viscosity; increasing return lymphatic flow whereby an antiedematous effect is obtained.

After oral administration, calcium dobesilate is fully absorbed by gastrointestinal tract, reaching 8 mcg/ml plasma peak around 6 hours.

Plasma half-life of calcium dobesilate is about 5 hours, linked to plasma proteins in about 20 to 25%. 50% from administered dose is removed in urine within the first 24 hours. The remaining 50% is removed in feces.

There are several pharmaceutical products in marketplace which are directed for chronic venous insufficiency treatment, wherein several active principles which are independently formulated are found; however, in this kind of pathology it is quite important to prevent severe complications, as well as rapidly reducing symptomatology in patients who suffer from CVI; therefore, the activity of a group of drugs which act through different ways is indispensable to reach a complete recovery for patients suffering said disease.

The pharmaceutical composition subject of the present invention comprises a synergistic combination of three active principles acting as venotonic and vasoprotective agents, such as active principle: Micronized Purified Flavonoid Fraction, equivalent to 90% Diosmine and 10% Hesperidin and active principle: Calcium dobesilate, in addition to pharmaceutically acceptable excipients, those which are formulated in a single dosage unit to be orally administered, which is indicated for control and treatment of chronic venous insufficiency. Said pharmaceutical composition has been developed taking into account that active principles comprised in formulation have a great efficacy and capacity for controlling and improving signs and symptoms manifested by said disease in shorter time, using lower concentrations thereof, in addition to achieve a maximization of therapeutic effect, an increase of its pharmacological action, a risk prevention of appearing severe complications and a reduction of risks of showing adverse effects.

The first venotonic and vasoprotective agent used in the pharmaceutical composition subject of the present invention, such as active principle: Diosmine, is present in the formulation in a concentration range from 225.0 mg to 450.0 mg per dose unit.

The second venotonic and vasoprotective agent used in the pharmaceutical composition subject of the present invention, such as active principle: Hesperidin, is present in the formulation in a concentration range from 25.0 mg to 50.0 mg per dose unit. The third venotonic and vasoprotective agent used in the pharmaceutical composition subject of the present invention, such as active principle: Calcium dobesilate, is present in the formulation in a concentration range from 250.0 mg to 1.0 g per dose unit.

In order to assess the efficiency of the pharmaceutical composition subject of the present invention, as well as the synergistic effect resulting from combining the active principles: Diosmine, Hesperidin and Calcium dobesilate in a single dosage unit, a comparative clinical study was carried out whereby above mentioned active principles were separately administered, as well as a combination thereof.

### COMPARATIVE STUDY ON DIOSMINE / HESPERIDIN EFFICIENCY; CALCIUM DOBESILATE VS A COMBINATION OF THE THREE AGENTS IN PATIENTS WITH CHRONIC VENOUS INSUFFICIENCY.

A randomized, comparative clinical study was carried out wherein 150 patients with chronic venous insufficiency were included.

All patients were firstly treated with conventional therapy, combining compression with pharmacological treatment.

Patients were divided in three treatment groups:
- Patients in Group 1: received Diosmine 450 mg / Hesperidin 50 mg every 12 hours, along two months.
- Patients in Group 2: received calcium dobesilate 500 mg every 12 hours, along two months.
- Patients in Group 3: They received a combination of Diosmine 225 mg / Hesperidin 25 mg / Calcium dobesilate 250 mg once a day, during two months.

A full clinical history with physical examination was prepared for the patients, being assessed with Echo Doppler and CEAP classification, see Table 1.

**Table 1 CEAP Analysis**

| | |
|---|---|
| "C" | letter assesses clinical findings |
| C0 | No visible or palpable signs of venous lesion are present |
| C1 | Presence of telangiectasies or reticular veins |
| C2 | Varices |
| C3 | Edema |
| C4 | Cutaneous changes related with venous pathology, without ulceration |
| C5 | Cutaneous changes with healed ulcer |
| C6 | Cutaneous changes with active ulcer |
| | After the number the "A" letter is written when patient is asymptomatic and "S" when symptoms are shown |
| | |
| "E" | letter refers to etiology |
| Ec | Congenital disease |
| Ep | Primary disease or without known cause |
| Es | Secondary disease or with known cause |
| | |
| "A" | letter discloses anatomical findings found with Echo-Doppler |
| | Surface veins (As)* |
| | Deep veins (Ap)* |
| | Perforating veins |
| "P" | letter refers to physiopathology |
| PR | Reflux |
| PO | Obstruction |
| PR, O | Both |

| | |
|---|---|
| * A number is added as a function of the affected vein | |

### RESULTS

There were not significant differences among groups as to age and sex.

20 patients from Group 1 and 14 from Group 2, reported side effects such as: head ache and tiredness sensation; however, treatment was not required to be cancelled. There were no adverse effects in Group 3, see Table 2.

**Table 2. Initial demographic and symptomatologic data**

| | Group 1 | Group 2 | Group 3 |
|---|---|---|---|
| | (n=50) | (n=50) | (n=50) |
| Age | 45 ± 9 | 46 ± 5 | 47 ± 7 |
| Sex F/M | 48 / 2 | 49 / 1 | 46 / 4 |
| Limb pain | 100% | 100% | 100% |
| Heaviness | 100% | 100% | 100% |
| Itch | 80% | 83% | 88% |
| Doppler | >9 | >9 | >9 |
| Cramps | 100% | 100% | 100% |

All patients who were included within the 3 treatment groups showed venous injury visible or palpable alterations, in Table 3 shown as 0.

All patients who were included in these 3 groups have the presence of telangiectasies or reticular veins (C1). Moreover, 100% of patients showed varices (C2).

None of the patients included within the 3 groups showed edema at the beginning of the visit (C3).

100% of patients showed cutaneous changes related with pathology such as: pigmentation and lipodermatosclerosis, without ulceration (C4).

None of the patients has cutaneous changes with healed ulcer or active ulcer (C5 and C6).

2 patients from Group 1, as well as 4 patients from Group 2 and 4 patients from Group 3 showed primary etiology or without any known cause (Ep).

Other patients in the three groups showed secondary disease or disease with known cause, mostly post-traumatism (Es).

Deep veins (Ap) were mostly affected.

Physiopathology demonstrated that it was caused by reflux (PR).

### CONCLUSIONS.

Side effects reported in Groups 1 and 2, correspond to a symptomatology due to venous dilatation and a better venous return; however, treatment was not required to be cancelled.

A significant and important change in patient symptomatology may be observed in Tables 2 and 4; although in the three groups there was a full symptomatology improvement, it is remarkable that under Group 3 the combination showed similar results compared with those from Groups 1 and 2 by using lower concentrations of active principles, without side effect manifestation. The use of lower concentrations of combined active principles produces an efficient synergistic effect with lower risks that side effects are present.

**Table 3. Initial CEAP analysis showed the following data**

| | Group 1 | Group 2 | Group 3 |
|---|---|---|---|
| | (n=50) | (n=50) | (n=50) |
| C0 | 0 | 0 | 0 |
| C1s | 100% | 100% | 100% |
| C2s | 100% | 100% | 100% |
| C3 | 0 | 0 | 0 |
| C4s | 100% | 100% | 100% |
| C5 | 0 | 0 | 0 |
| C6 | 0 | 0 | 0 |
| Ec | 0 | 0 | 0 |
| Ep | 2 | 4 | 4 |
| Es | 48 | 46 | 46 |
| Veins | Ap | Ap | Ap |
| PR | 100% | 100% | 100% |

| | | | |
|---|---|---|---|
| C=Clinical findings E=Etiology A=Anatomical findings P= Physiopathology | | | |

**Table 4. Final symptomatology data**

| | Group 1 | Group 2 | Group 3 |
|---|---|---|---|
| | (n=50) | (n=50) | (n=50) |
| Limb pain | 0 | 0 | 0 |
| Heaviness | 0 | 0 | 0 |
| Itch | 0 | 0 | 0 |
| Doppler | <9 | <9 | <9 |
| Cramps | 0 | 0 | 0 |

## Claims

1. A pharmaceutical composition suitable for control and treatment of chronic venous insufficiency **characterized in that** comprises a synergistic combination of Diosmine, Hesperidin and Calcium dobesilate in addition to pharmaceutically acceptable excipients.

2. A pharmaceutical composition according to claim 1, **characterized in that** Diosmine is present in the formulation in a concentration of 225.0 mg, Hesperidin in a concentration of 25.0 mg and Calcium dobesilate in a concentration of 250.0 mg.

3. A pharmaceutical composition according to claims 1 and 2, **characterized in that** is formulated in a single dosage unit.

4. A pharmaceutical composition according to claims 1 to 3, **characterized in that** it is formulated to be orally administered in capsule or tablet form.

5. The use of the pharmaceutical composition according to claims 1 to 4 in the manufacture of a medicament for control and treatment of chronic venous insufficiency.

## Patentansprüche

1. Eine pharmazeutische Zusammensetzung, welche zur Kontrolle und Behandlung von chronischer Veneninsuffizienz geeignet ist, **dadurch gekennzeichnet, dass** sie neben pharmazeutisch verträglichen Hilfsstoffen eine synergistische Kombination von Diosmin, Hesperidin und Calciumdobesilat umfasst.

2. Eine pharmazeutische Zusammensetzung nach Anspruch 1, **dadurch gekennzeichnet, dass** in der Formulierung Diosmin in einer Konzentration von 225,0 mg, Hesperidin in einer Konzentration von 25,0 mg und Calciumdobesilat in einer Konzentration von 250,0 mg vorliegt.

3. Eine pharmazeutische Zusammensetzung nach Anspruch 1 und 2, **dadurch gekennzeichnet, dass** sie in einer Einzeldosiseinheit formuliert ist.

4. Eine pharmazeutische Zusammensetzung nach Ansprüchen 1 bis 3, **dadurch gekennzeichnet, dass** sie zur oralen Verabreichung in Kapsel- oder Tablettenform formuliert ist.

5. Verwendung der pharmazeutischen Zusammensetzung nach Ansprüchen 1 bis 4 in der Herstellung eines Arzneimittels zur Kontrolle und Behandlung von chronischer Veneninsuffizienz.

## Revendications

1. Une composition pharmaceutique appropriée pour le contrôle et traitement de l'insuffisance veineuse chronique, **caractérisée en ce qu'**elle comprend une combinaison synergique de Diosmine, d'Hespéridine et du Dobésilate de calcium ainsi que des excipients pharmaceutiquement acceptables.

2. Composition pharmaceutique selon la revendication 1, **caractérisée en ce que** la Diosmine est présente dans la formulation en une concentration de 225,0 mg, l'Hespéridine en une concentration de 25,0 mg et le Dobésilate de calcium en une concentration de 250,0 mg.

3. Composition pharmaceutique selon les revendications 1 et 2, **caractérisée en ce qu'**elle est formulée en une seule unité de dosage.

4. Composition pharmaceutique selon les revendications 1 à 3, **caractérisée en ce qu'**elle est formulée pour être administrée oralement sous la forme de capsules ou de comprimés.

5. Utilisation de la composition pharmaceutique selon les revendications 1 à 4 dans la fabrication d'un médicament pour le contrôle et traitement de l'insuffisance veineuse chronique.
